# EUROPÄISCHE PATENTSCHRIFT

(11) **EP 2 089 715 B1**
(45) Veröffentlichungstag und Bekanntmachung des Hinweises auf die Patenterteilung: **08.08.2012**
(21) Anmeldenummer: 07815155.2
(22) Anmeldetag: 19.10.2007
(51) Int. Cl.: G01N 33/573

(54) **VERFAHREN ZUR BESTIMMUNG DER AKTIVITÄT VON ACE2**
METHOD FOR DETERMINING ACE2 ACTIVITY
PROCÉDÉ DE DÉTERMINATION DE L'ACTIVITÉ DE L'ECA2

(30) Priorität: 19.10.2006 AT 17582006
(43) Veröffentlichungstag der Anmeldung: 19.08.2009
(73) Patentinhaber: Apeiron Biologics AG, 1030 Wien (AT)
(72) Erfinder: LOIBNER, Hans, 1230 Wien (AT); SCHUSTER, Manfred, 2191 Schrick (AT); JANZEK-HAWLAT, Evelyne, 1230 Wien (AT)
(74) Vertreter: Sonn & Partner Patentanwälte
(86) Internationale Anmeldenummer: PCT/AT2007/000488
(87) Internationale Veröffentlichungsnummer: WO 2008/046125

(56) Entgegenhaltungen:
- JP-A- 8 160 046
- US-A1- 2005 287 066
- US-B1- 6 610 497
- GUY JODIE L ET AL: "Angiotensin-converting enzyme-2 (ACE2): Comparative modeling of the active site, specificity requirements, and chloride dependence." BIOCHEMISTRY, Bd. 42, Nr. 45, 18. November 2003 (2003-11-18), Seiten 13185-13192, XP002464544 ISSN: 0006-2960
- VICKERS CHAD ET AL: "Hydrolysis of biological peptides by human angiotensin-converting enzyme-related carboxypeptidase" JOURNAL OF BIOLOGICAL CHEMISTRY, Bd. 277, Nr. 17, 26. April 2002 (2002-04-26), Seiten 14838-14843, XP002464545 ISSN: 0021-9258 in der Anmeldung erwähnt

### SEQUENZPROTOKOLL

<110> Apeiron Biologics Forschungs- und Entwicklungsgesellschaft m.b.H.
<120> Verfahren zur Bestimmung der Aktivität von ACE2
<130> r50504
<150> AT A 1758/2006
   <151> 2006 10 19
<160> 2
<170> PatentIn version 3.3
<210> 1
   <211> 740
   <212> PRT
   <213> Homo sapiens
<400> 1
   Met Ser Ser Ser Ser Trp Leu Leu Leu Ser Leu Val Ala Val Thr Ala
   1               5                   10                  15
   Ala Gln Ser Thr Ile Glu Glu Gln Ala Lys Thr Phe Leu Asp Lys Phe
               20                  25                  30
   Asn His Glu Ala Glu Asp Leu Phe Tyr Gln Ser Ser Leu Ala Ser Trp
           35                  40                  45
   Asn Tyr Asn Thr Asn Ile Thr Glu Glu Asn Val Gln Asn Met Asn Asn
       50                  55                  60
   Ala Gly Asp Lys Trp Ser Ala Phe Leu Lys Glu Gln Ser Thr Leu Ala
   65                  70                  75                  80
   Gln Met Tyr Pro Leu Gln Glu Ile Gln Asn Leu Thr Val Lys Leu Gln
                   85                  90                  95
   Leu Gln Ala Leu Gln Gln Asn Gly Ser Ser Val Leu Ser Glu Asp Lys
               100                 105                 110
   Ser Lys Arg Leu Asn Thr Ile Leu Asn Thr Met Ser Thr Ile Tyr Ser
           115                 120                 125
   Thr Gly Lys Val Cys Asn Pro Asp Asn Pro Gln Glu Cys Leu Leu Leu
       130                 135                 140
   Glu Pro Gly Leu Asn Glu Ile Met Ala Asn Ser Leu Asp Tyr Asn Glu
   145                 150                 155                 160
   Arg Leu Trp Ala Trp Glu Ser Trp Arg Ser Glu Val Gly Lys Gln Leu
                   165                 170                 175
   Arg Pro Leu Tyr Glu Glu Tyr Val Val Leu Lys Asn Glu Met Ala Arg
               180                 185                 190
   Ala Asn His Tyr Glu Asp Tyr Gly Asp Tyr Trp Arg Gly Asp Tyr Glu
   195 200 205
   Val Asn Gly Val Asp Gly Tyr Asp Tyr Ser Arg Gly Gln Leu Ile Glu
   210 215 220
   Asp Val Glu His Thr Phe Glu Glu Ile Lys Pro Leu Tyr Glu His Leu
   225 230 235 240
   His Ala Tyr Val Arg Ala Lys Leu Met Asn Ala Tyr Pro Ser Tyr Ile
   245 250 255
   Ser Pro Ile Gly Cys Leu Pro Ala His Leu Leu Gly Asp Met Trp Gly
   260 265 270
   Arg Phe Trp Thr Asn Leu Tyr Ser Leu Thr Val Pro Phe Gly Gln Lys
   275 280 285
   Pro Asn Ile Asp Val Thr Asp Ala Met Val Asp Gln Ala Trp Asp Ala
   290 295 300
   Gln Arg Ile Phe Lys Glu Ala Glu Lys Phe Phe Val Ser Val Gly Leu
   305 310 315 320
   Pro Asn Met Thr Gln Gly Phe Trp Glu Asn Ser Met Leu Thr Asp Pro
   325 330 335
   Gly Asn Val Gln Lys Ala Val Cys His Pro Thr Ala Trp Asp Leu Gly
   340 345 350
   Lys Gly Asp Phe Arg Ile Leu Met Cys Thr Lys Val Thr Met Asp Asp
   355 360 365
   Phe Leu Thr Ala His His Glu Met Gly His Ile Gln Tyr Asp Met Ala
   370 375 380
   Tyr Ala Ala Gln Pro Phe Leu Leu Arg Asn Gly Ala Asn Glu Gly Phe
   385 390 395 400
   His Glu Ala Val Gly Glu Ile Met Ser Leu Ser Ala Ala Thr Pro Lys
   405 410 415
   His Leu Lys Ser Ile Gly Leu Leu Ser Pro Asp Phe Gln Glu Asp Asn
   420 425 430
   Glu Thr Glu Ile Asn Phe Leu Leu Lys Gln Ala Leu Thr Ile Val Gly
   435 440 445
   Thr Leu Pro Phe Thr Tyr Met Leu Glu Lys Trp Arg Trp Met Val Phe
   450 455 460
   Lys Gly Glu Ile Pro Lys Asp Gln Trp Met Lys Lys Trp Trp Glu Met
   465 470 475 480
   Lys Arg Glu Ile Val Gly Val Val Glu Pro Val Pro His Asp Glu Thr
   485 490 495
   Tyr Cys Asp Pro Ala Ser Leu Phe His Val Ser Asn Asp Tyr Ser Phe
   500 505 510
   Ile Arg Tyr Tyr Thr Arg Thr Leu Tyr Gln Phe Gln Phe Gln Glu Ala
   515 520 525
   Leu Cys Gln Ala Ala Lys His Glu Gly Pro Leu His Lys Cys Asp Ile
   530 535 540
   Ser Asn Ser Thr Glu Ala Gly Gln Lys Leu Phe Asn Met Leu Arg Leu
   545 550 555 560
   Gly Lys Ser Glu Pro Trp Thr Leu Ala Leu Glu Asn Val Val Gly Ala
   565 570 575
   Lys Asn Met Asn Val Arg Pro Leu Leu Asn Tyr Phe Glu Pro Leu Phe
   580 585 590
   Thr Trp Leu Lys Asp Gln Asn Lys Asn Ser Phe Val Gly Trp Ser Thr
   595 600 605
   Asp Trp Ser Pro Tyr Ala Asp Gln Ser Ile Lys Val Arg Ile Ser Leu
   610 615 620
   Lys Ser Ala Leu Gly Asp Lys Ala Tyr Glu Trp Asn Asp Asn Glu Met
   625 630 635 640
   Tyr Leu Phe Arg Ser Ser Val Ala Tyr Ala Met Arg Gln Tyr Phe Leu
   645 650 655
   Lys Val Lys Asn Gln Met Ile Leu Phe Gly Glu Glu Asp Val Arg Val
   660 665 670
   Ala Asn Leu Lys Pro Arg Ile Ser Phe Asn Phe Phe Val Thr Ala Pro
   675 680 685
   Lys Asn Val Ser Asp Ile Ile Pro Arg Thr Glu Val Glu Lys Ala Ile
   690 695 700
   Arg Met Ser Arg Ser Arg Ile Asn Asp Ala Phe Arg Leu Asn Asp Asn
   705 710 715 720
   Ser Leu Glu Phe Leu Gly Ile Gln Pro Thr Leu Gly Pro Pro Asn Gln
   725 730 735
   Pro Pro Val Ser
   740
<210> 2
   <211> 13
   <212> PRT
   <213> Homo sapiens
<400> 2
   Glu Phe Leu Gly Ile Gln Pro Thr Leu Gly Pro Pro Asn
   1               5                   10

## Beschreibung

Die vorliegende Erfindung betrifft die immunologische Bestimmung der enzymatischen Aktivität von Enzymen.

Derzeitige Methoden zur Bestimmung von Enzymaktivitäten in raschen Screeningverfahren werden stark von interferierenden Faktoren von diversen Quellen beeinflusst. Übliche Assays zur Messung von Enzymaktivitäten sehen die Verwendung von fluoreszierenden oder chromogenen Verbindungen vor. Beispielsweise wird bei der Detektion von HIV Protease ein fluoreszierendes Substrat verwendet, das mit einer fluorezierenden Dansyl-Gruppe an einem Ende des Peptids und einem Fluoreszenzquencher am anderen Ende des Peptids modifiziert wurde. Ein Anstieg an gemessenem Fluoreszenzsignal erfolgt durch Spaltung des Peptids durch die Protease, da der Emitterteil vom unterdrückenden Quencher geteilt wird. Fluoreszierende Substrate für andere Enzyme können durch ähnliche Modifizierungen der Substrate des jeweiligen Enzyms modifiziert werden, wie beispielsweise in der US 6,037,137, EP 1 557 474 A und WO 90/00618 offenbart werden.

Diese Assays, insbesondere im High-throughput Bereich, neigen zu Problemen mit Autofluoreszenz von biologischen Komponenten, zum Teil bedingt durch die gemessenen Moleküle selbst. Viele der Verbindungen und natürlichen Extrakte sind selbst farbig oder fluoreszieren und befinden sich während der Signalmessung des Assays in Lösung. Das führt dazu, dass diese Interferenz die Detektion und die Sensitivität bzw. den dynamischen Messbereich der Methode limitiert. Die Interferenz kann auch als Inhibition des Enzyms interpretiert werden, wobei es in Inhibitionsassays schwer ist, die gemessene Inhibition von der tatsächlichen (bio)chemischen Inhibition des Enzyms zu unterscheiden.

Die US 5,591,591 beschreibt einen Assay zur Detektion von Proteasen, wobei eine Dioxetan-Komponente mit einer proteolytischen Enzym-spezifischen Aminosäure oder einem Peptid verbunden zu einer Probe die potentiell die Protease enthält, zugefügt wird, wobei die Aminosäure durch die enzymatische Reaktion entfernt wird, was wiederum die Zersetzung des Dioxetans und Chemilumineszenz bewirkt.

Die JP 8160046 betrifft die Verwendung eines Kits zur Bestimmung der ACE-Aktivität.

Die WO 2002/098448 A1 betrifft Verbindungen, die spezifisch ACE2 binden und dessen Aktivität verändern.

Die US 2003/0124622 beschreibt die Messung der Aktivität einer Protease, wobei die Protease zunächst an einen immobilisierten Antikörper gebunden wird und ein chromogenes Substrat zugegeben wird, das sich unter der Einwirkung der Enzymaktivität verfärbt. Die Änderung der Extinktion kann mit der Enzymaktivität korreliert werden.

Die US 6,610,497 beschreibt die Isolierung von ACE2 und nennt auch eine Vielzahl an Möglichkeiten zur Bestimmung von ACE2 bzw. ACE2-Bindungspartnerkomplexen.

Die US 2005/0287066 A1 betrifft monoklonale Antikörper die die N-Domäne von ACE, also nicht ACE2, spezifisch binden.

Guy et al. (Biochemistry 42 (45)(2003): 13185-13192) beschreiben die Aktivität und Spezifität von ACE2.

Vickers et al. (Biological Chemistry 277 (17)(2002): 14838-14843) nennen Hintergrundinformationen zu ACE2 und beschreiben auch Substrate zur ACE2-Aktivitätsmessung.

Es ist ein Ziel schnelle Messsysteme zu entwickeln, die von Verunreinigungen unbeeinflusst sind, insbesondere für Systeme zur raschen Messung von Enzymaktivitäten.

Die vorliegende Erfindung betrifft ein Verfahren zur Bestimmung der Aktivität von ACE2, eines Enzyms des Renin-Angiotensin-Aldosteron-Systems, umfassend folgende Schritte:
- zur Verfügung stellen von an einen festen Träger immobilisierten ACE2-bindenden Einheiten, die spezifisch für einen Teil des ACE2 sind, der nicht an der katalytischen Aktivität des ACE2 beteiligt ist,
- Kontaktieren der immobilisierten ACE2-bindenden Einheiten mit einer Probe, die potentiell ACE2 enthält, wobei ACE2 von der ACE2-bindenden Einheit gebunden wird,
- Entfernen der nicht-bindenden Anteile der Probe von dem an die ACE2-bindenden Einheiten gebundenen Enzym,
- Zufügen eines Substrates des ACE2, das durch die Enzymaktivität umgesetzt wird und die Umsetzung ein Signal liefert,
- Messen der Änderung des Signals in einer bestimmten Zeitspanne, wobei die Änderung mit der ACE2-Aktivität korreliert werden kann.

Mit dem erfindungsgemäßen Verfahren ist es möglich, quantitativ die enzymatische Aktivität in komplexen Lösungen, wie Körperflüssigkeiten oder Kulturüberständen, zu bestimmen unter Verwendung der endogenen Aktivität des zu quantifizierenden Enzyms unter Umsetzung eines Signal-liefernden Substrates.

Zur direkten Bestimmung von aktiven Enzymen in komplexen Lösungen, wie Körperflüssigkeiten, wurde eine Methode aufgebaut, welche zwei wesentliche Schritte kombiniert und einen hohen Probendurchsatz erlaubt: Die immunologische Bindung des Enzyms an einen Träger, wie beispielsweise eine Mikrotiterplatte, wodurch die Abreicherung sämtlicher anderer Komponenten erreicht wird, gefolgt von einer spezifischen enzymatischen Reaktion auf Basis eines fluoreszenzmarkierten Substrates, welches ein messbares Signal liefert.

Im ersten Schritt wird die komplexe Lösung z.B. in einer Mikrotiterplatte inkubiert, in der zuvor z.B. ein ACE2-spezifischer Antikörper immobilisiert wurde. Ein solcher Antikörper erkennt vorzugsweise einen Teil des Enzyms, der nicht an der katalytischen Reaktion beteiligt ist, und beeinträchtigt nicht die enzymatische Reaktion. Wenn der Antikörper hingegen Regionen des Enzyms erkennt, die in die enzymatische Reaktivität direkt involviert sind, wird die Empfindlichkeit des Assays herabgesetzt bzw. der Assay funktioniert nicht mehr, da die Enzymaktivität blockiert ist. Sämtliche weitere Bestandteile der komplexen Probe (z.B. Serum) binden nicht an den festen Träger, z.B. eine Plattenoberfläche, und werden in einem nachfolgenden Schritt aus dem System entfernt, vorzugsweise von der Platte gewaschen. Waschschritte können gegebenfalls wiederholt werden, z.B. 1, 2, 3 oder mehrere Male. Letztendlich verbleibt ausschließlich korrekt gebundenes Enzym auf der Platte. Nach Zugabe eines fluoreszenzmarkierten Substrates, welches durch das Enzym gespalten wird und das im gespaltenen Zustand eine deutlich höhere Fluoreszenz aufweist, wird nach einer definierten Inkubationszeit die Fluoreszenz gemessen und mit einem Standard bekannter Aktivität und Konzentration verglichen. Üblicherweise wird dies durch Zweikomponenten-Fluoreszenz-Quenching-Systeme erzielt, wobei die Fluoreszenz durch Abspaltung des Quenchers erhöht wird.

Durch die Einwirkung bzw. Umsetzung des Enzyms auf das Substrat wird dieses vorzugsweise in der Extinktion oder Fluoreszenz verändert, die mit optischen Methoden gemessen werden kann. Chromogene Substrate die zu einem solchen Zweck modifiziert werden können um ein Änderung der Extinktion (oder Absorption) zu verfolgen sind beispielsweise aus der US 2003/0124622 bekannt. Im weiteren Ausführungsformen weist das Substrat einen fluoreszierenden Teil und einen Fluoreszenzquencherteil auf die durch die Enzymaktivität getrennt werden können. Durch die Umsetzung durch das Enzym kann daher eine Änderung der Fluoreszenz gemessen und verfolgt werden.

Die ACE2-bindende Einheit ist vorzugsweise ein Antikörper oder ein künstlicher oder natürlicher Rezeptor des Enzyms, der die Aktivität von ACE2 nicht verhindert.

Das erfindungsgemäß bestimmte Enzym ist ACE2, eine Peptidase oder Protease des Renin-Angiotensin-Aldosteron-Systems (RAAS) - auch als Renin-Angiotensin-System (RAS) bezeichnet. Die proteolytische Aktivität des RAS stellt eine Kaskade von enzymatischen Schritten zur Regulierung des Blutdrucks dar. ACE (Angiotensin Converting Enzyme) ist hierin das wohl bekannteste Enzym, dass Angiotensin I zu Angiotensin II umsetzt. Angiotensin II wirkt blutdrucksteigernd, weswegen ACE-Hemmer häufig zur Behandlung von erhöhtem Blutdruck eingesetzt werden. Unmittelbar nach der Entdeckung von ACE2 wurde angenommen, dass es die gleiche Aktivität wie ACE aufweisen sollte (US 6,194,556), weswegen es nach diesem Enzym benannt wurde. Sowohl ACE als auch ACE2 sind Metalloproteasen mit einem katalytischen Zink-Atom im Zentrum. Diese ursprüngliche Annahme stellte sich jedoch als Irrtum heraus. Sowohl von der Aktivität her, als auch mechanistisch sind ACE und ACE2 völlig unterschiedlich. Während ACE Blutdruck-steigernd wirkt, bewirkt ACE2 eine Blutdrucksenkung (1). Mechanistisch ist ACE eine Peptidyl-Dipeptidase, ACE2 hingegen eine Carboxypeptidase (9). Somit sind ACE und ACE2 zwei völlig verschiedene Enzyme mit unterschiedlichen Voraussetzungen zur Messung ihrer Aktivität.

ACE2 (Angiotensin Converting Enzyme 2) stellt ein Schlüsselenzym des Renin-Angiotensin-Systems dar. Als Carboxypeptidase wird es membranverankert (mACE2), als Rezeptor vor allem auf Lungen-, Nieren- und Herzzellen exprimiert und spaltet diverse Peptidsubstrate. ACE2 kommt jedoch auch als lösliches Protein (sekretiertes ACE2, seACE2) ohne Transmembrandomäne und cytoplasmischem Anteil in Körperflüssigkeiten vor. Prominente Substratvertreter sind Apellin, Bradykinin aber ebenso Angiotensin-I, welches zu Angiotensin 1-9, oder Ang-II, welches zu Ang 1-7 gespalten wird (1, 2). ACE2 ist maßgeblich für die Homeostasis des Organismus verantwortlich (3).

Apeiron Biologics entwickelte lösliches ACE2 als Therapeutikum zur Behandlung von ARDS oder ALI, zwei akuten Lungenerkrankungen, mit welchen als Begleitsymptom ein herunter regulierter ACE2 Titer einhergeht (4, 5), wie mehrere Studie bestätigen. Allerdings ist durchaus auch vorgesehen, dass lösliches ACE2 auch zur Behandlung von Herz- oder Nierenerkrankungen eingesetzt wird (1, 6). Nachdem ACE2 in all diesen Erkrankungen eine wesentliche Rolle spielt, liefert eine robuste Bestimmungsmethode zur Quantifizierung von löslichem, aktiven ACE2 wichtige Erkenntnisse in der Erforschung dieser Indikationen. Ferner bietet sich eine solche Methode zur Ermittlung der Pharmakokinetik und Pharmakodynamik einer Therapie mit löslichem ACE2 an. Der Gehalt an enzymatisch aktivem ACE2 in Körperflüssigkeiten stellt außerdem ein Schlüsselergebnis dar, welches den Zugriff auf diverse therapeutische und diagnostische Bereiche eröffnet.

Klassische, immunologische Bestimmungsmethoden (ELISA) von ACE2-Konzentrationen liefern nur ein unvollständiges Resultat, da diese Methoden sowohl aktives, als auch enzymatisch inaktives Enzym erfassen können. Aufschluss über die Funktion von ACE2 kann aber ausschließlich durch Bestimmung des enzymatisch aktiven Anteils erlangt werden. Die Bestimmung der Aktivität von Peptidasen in komplexen Flüssigkeiten, wie Vollblut, Serum oder Plasmaproben, erwies sich bislang jedoch im Stand der Technik als schwierig, weil zahlreiche Komponenten dieser komplexen Lösungen die enzymatische Reaktion beeinflussen können. Inhibierende Bestandteile sowie Peptidasen geringer Spezifität können den gemessenen Substratumsatz in beide Richtungen verfälschen, was einen Aktivitätsbestimmungsansatz direkt aus diesen komplexen Systemen üblicherweise verhindert. Insbesondere für ACE2 konnte daher bislang auch kein zufriedenstellendes System zur Verfügung gestellt werden, welches vor allem auch der Komplexität der Proben, in denen ACE2 gemessen werden kann, Rechnung trägt.

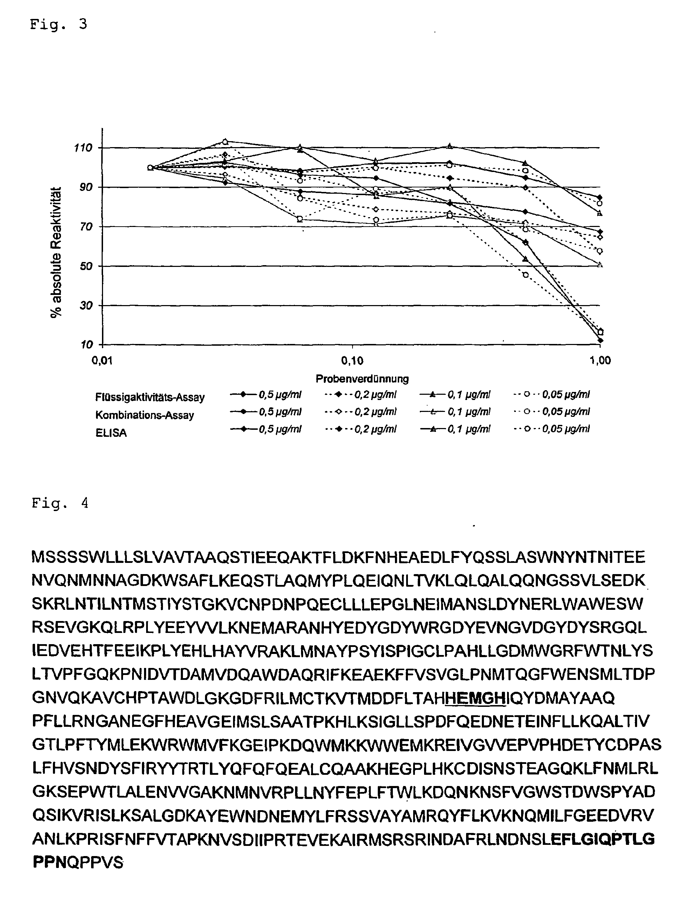

Besonders bevorzugt ist ACE2 ausgewählt aus Membran-gebundenem ACE2 (mACE2) und sekretiertem ACE2 (seACE2), wobei vorzugsweise die Enzym-bindende Einheit spezifisch für den C-terminalen Teil der extrazellulären Domain von ACE2 ist. ACE2, welches noch mit der Membrandomäne durch Hilfe von Detergentien in Lösung gehalten wird, sollte den C-terminalen Teil aufgrund der Nähe der hydrophoben Region deutlich geringer in die hydrophile Lösung exponieren. Die Sequenz von ACE2 ist in Fig. 4 (SEQ ID NO: 1) angegeben, wobei das aktive Zentrum - Zink-bindende-Motif HEXXH durch Unterstreichen gekennzeichnet ist. Das erfindungsgemäße Verfahren funktioniert, wenn das gebundene ACE2 noch Enzymaktivität hat. Gemäß der Sequenz ist die ACE2-bindende Einheit für eine Teilsequenz von mindestens 4, vorzugsweise mindestens 5 oder 6, besonders bevorzugt mindestens 7, speziell bevorzugt mindestens 8 aufeinander folgenden Aminosäuren der 373 N-terminalen Aminosäuren von ACE2 oder der 362 C-terminalen Aminosäuren von ACE2 spezifisch. In besonderen Ausführungsformen ist diese Teilsequenz aus den 360, bevorzugt 340 oder 320 N-terminalen oder C-terminalen Aminosäuren ausgewählt. Eine solche Sequenz, gegen die eine ACE2-bindende Einheit gerichtet werden kann, ist z.B. EFLGIQPTLGPPN (SEQ ID NO:2).

Des Weiteren ist die Kristallstruktur von ACE2 veröffentlicht (9) und als natives Enzym (PDB-Datenbank-Eintrag: 1R42) und als Inhibitor-gebundenes Enzym bekannt (PDB-Datenbank-Eintrag: 1R4L). Diese native Struktur zeigt als Mittelpunkt des aktiven Zentrums ein Zn-Atom und dient im folgenden als Referenz für ACE2. Eine weitere Kristallstruktur ist beispielsweise unter dem PDB-Datenbank-Eintrag 108A (natives menschliches ACE) bekannt, in der ebenfalls ein katalytisches Zink-Atom ersichtlich ist. Bevorzugt ist die Enzym-bindende Einheit für einen Teil von mindestens 4, 5, 6, 7 oder 8 Aminosäuren des Enzyms spezifisch, der einen Mindestabstand von 0,5 nm, vorzugsweise 0,6 nm, besonders bevorzugt 0,7 nm oder 0,8 nm, insbesondere 0,9 nm oder 1,0 nm vom aktiven Zentrums aufweist (gemäß der Kristallstruktur des Enzyms). Hierzu kann der geometrische Mittelpunkt der Teilsequenz hinzugezogen werden - wobei die Teilsequenz nicht unbedingt aus sequentiell aufeinanderfolgenden Aminosäuren bestehen muss, da z.B. Antikörper nicht Sequenzen sondern räumliche Strukturen erkennen. Zur Ermittlung der Position des aktiven Zentrums kann der Mittelwert der Koordinaten der Aminosäuren hinzugezogen werden oder die Position des katalytischen Metallions, die an der Enzymreaktion beteiligt sind.

Die Teile oder Teilsequenzen des ACE2 können in bestimmten Ausführungsformen bis zu 10, bis zu 12, bis zu 15, bis zu 20, bis zu 30 oder auch bis zu 40 oder 50 Aminosäuren des Enzyms aufweisen.

Eine ACE2-bindende Einheit ist vorzugsweise ein Antikörper, der den C-terminalen Teil der extrazellulären Domaine von natürlichem ACE2 bindet. Dies führt somit zu einer gerichteten Präsentation des Enzyms auf der Plattenoberfläche, in Analogie zur Präsentation an der Zelloberfläche sind ohne die ACE2-Aktivität empfindlich zu beeinflussen.

Aktivitätsbestimmungsverfahren können sowohl fluoreszierende Substrate oder chromogene Substrate verwendet werden, wobei fluoreszierende aufgrund der Sensitivität bei Blutproben bevorzugt werden.

Im erfindungsgemäßen Verfahren sind die ACE2-bindenden Einheiten bevorzugt ausgewählt aus Antikörpern, Antikörperfragmenten oder Antikörperderivaten. Solche Einheiten beinhalten Fv, Fab oder F(ab)₂-Teile von Antikörpern sowie Einzelkettenantikörper (single chain antibodies, SCA), wie in der US 4,946,778 offenbart, oder Einzelketten-Antigen-bindende Fragmente (ScFv).

In speziellen Ausführungsformen sind die Antikörper, -fragmente oder -derivate polyklonal, wobei, vorzugsweise Antikörper, -fragmente oder -derivate, die für die aktiven Zentren des Enzyms spezifisch sind, auf unter 20% bevorzugt auf unter 10%, mehr bevorzugt auf unter 5%, am meisten bevorzugt auf unter 1% abgereichert sind - z.B. durch Immunoabsorption, wobei Teile mit aktiven Zentren der Enzyme immobilisiert präsentiert werden und das Eluat die abgereicherten, bevorzugten Antikörper enthält.

In anderen Ausführungsformen sind die Antikörper, -fragmente oder -derivate monoklonal. Hierbei werden spezielle Antikörper selektiert, die die ACE2-Aktivität unbeeinflusst (oder gering verändert) lassen. Bei bestimmten Enzymen können Aktivitätseinbußen durch die Antikörperbindung nicht vermieden werden. Hierbei ist es jedoch mit gängigen Auswahlverfahren (z.B. Phagendisplay-Methoden) möglich, diese Inhibierung niedrig zu halten. So wird bevorzugt die enzymatische Aktivität nicht mehr als 30%, bevorzugt nicht mehr als 20% besonders bevorzugt nicht mehr als 10%, speziell bevorzugt nicht mehr als 5%, am meisten bevorzugt nicht mehr als 1% durch den Antikörper, das -derivat oder das -fragment inhibiert. Besonders bevorzugte Antikörper können die enzymatische Aktivität sogar erhöhen.

Das Substrat, das erfindungsgemäß verwendet wird, ist bevorzugt ein niedermolekulares Peptid mit einer Länge von 1 bis 30 Aminosäuren, bevorzugt 1 bis 20 Aminosäuren, insbesondere bevorzugt 1 bis 10 Aminosäuren ist, das von dem Enzym gespalten werden kann, und an einem zu erwartenden Spaltprodukt der Fluoreszenzteil und an einem anderen zu erwartenden Spaltprodukt der Quencher kovalent verbunden ist, bevorzugt Mca-Ala-Pro-Lys(Dnp)-OH (Mca: (7-methoxycoumarin-4-yl)acetyl) sofern das Enzym ACE2 ist. Andere Peptidsubstrate sind beispielsweise in (7) und (8) offenbart. Für ACE2 ist das Cumarin-di-Nitro-Phenyl System besonders geeignet. Andere Systeme werden beispielsweise in der US 6,037,137, der EP 1 557 474 A und der WO 90/00618 offenbart.

Die Probe kann chromophore und/oder fluoreszierende Verunreinigungen enthalten. Es ist ein wesentlicher Vorteil des erfindungsgemäßen Verfahrens, dass diese Störsubstanzen im Assay auf einfache Weise entfernt werden.

Bevorzugt ist die Probe ausgewählt aus Körperflüssigkeiten, homogenisierten fluiden Gewebeproben und Zellkulturüberständen, vorzugsweise aus Vollblut, Serum- oder Plasmaproben.

In einem weiteren Aspekt sieht die vorliegende Erfindung die Verwendung des erfindungsgemäßen Verfahrens zur in-vitro-Bestimmung von ACE2 Titern in Vollblut, Serum- oder Plasmaproben bzw. eines Enzyms in solchen Proben vor.

Ein anderer Aspekt betrifft ein Verfahren zur Diagnose eines Krankheitsanzeichens, das mit abnormer ACE2 Aktivität verbunden ist, welches dadurch gekennzeichnet ist, dass eine Probe eines Tieres oder Menschen zur Verfügung gestellt wird, und mit einem in-vitro-Verfahren nach der vorliegenden Erfindung die ACE2 Aktivität bestimmt wird, wobei eine Änderung der ACE2 Aktivität von über 40% oder 50%, vorzugsweise von über 50%, besonders bevorzugt von über 75%, im Vergleich zur ACE2 Aktivität im gesunden Zustand eine Krankheit oder die Gefährdung an einer Krankheit zu erkranken anzeigt. ACE2-indizierte Krankheitszustände können sowohl über einen Anstieg der ACE2-Aktivität angezeigt werden, z.B. Mykardinfarkt (10,11) oder über eine reduzierte ACE2-Aktivität, z.B. ARDS (4). Im Fall eines ACE2-Aktivitätsanstiegs ist die Aktivitätsänderung vorzugsweise mindestens 100%, insbesondere mindestens 150%. Der Aktivitätswert im gesunden Zustand hängt stark vom der Spezies und dem Gewebe ab und kann ebenfalls mit dem erfindungsgemäßen Verfahren bestimmt werden - z.B. als Durchschnittswert gesunder Organismen oder in einem nicht von der Krankheit betroffenen Referenzgewebe des selben Organismus'. Mit dem erfindungsgemäßen Verfahren können somit auf einfache und verlässliche Weise Anzeichen für eine Krankheit festgestellt bzw. eine Krankheit diagnostiziert werden.

Krankheiten, die erfindungsgemäß festgestellt oder angezeigt werden können, sind beispielsweise cardio-vaskuläre Erkrankungen (insbesondere Myokardinfarkt), Nierenerkrankungen und Lungenerkrankungen, insbesondere ARDS oder ALI. Spezielle ACE2-beeinflusste Lungenkrankheiten führen zur Ansammlung von Flüssigkeit in der Lunge, in der auch die ACE2-Aktivität bestimmt werden kann. In diesem Sinne ist unter den Körperflüssigkeitsproben eine Flüssigkeit in der Lunge bzw. in den Lungenbläschen explizit eingeschlossen.

Desweiteren beschreibt die vorliegende Druckschrift einen Kit (der zur Durchführung des erfindungsgemäßen Verfahrens geeignet ist), bestehend aus einem festen Träger mit immobilisierten Enzym-bindenden Einheiten, die spezifisch für einen Teil des Enzyms sind, der nicht an der katalytischen Aktivität des Enzyms beteiligt ist. Das Enzym ist, wie bereits oben beschrieben eine Protease oder Peptidase des RAS, vorzugsweise ACE2.

Bevorzugt umfasst der Kit weiters ein Substrat des ACE2, das einen fluoreszierenden Teil und einen Fluoreszenzquencherteil aufweist, die durch ACE2-Aktivität getrennt werden können.

Das immobilisierte ACE2 des Kits sowie die ACE2-bindenden Einheiten und das Substrat können wie oben beschrieben vorgesehen werden.

Die vorliegende Erfindung wird durch die folgende Figur und Beispiele näher erläutert ohne auf diese Ausführungen limitiert zu sein.
Figur 1: Immunologische Bestimmung der enzymatischen Aktivität von ACE2 in komplexen Lösungen: Lösungen unterschiedlicher ACE2- Konzentration (0 ng/ml: grau; 125 ng/ml: blau; 250 ng/ml: grün; 500 ng/ml: gelb und 1 µg/ml: rot) wurden nach immunologischer Anreicherung von ACE2 17 Stunden mit dem Peptidsubstrat Mca-Ala-Pro-Lys(Dnp)-OH inkubiert. Der Anstieg der Fluoreszenz wurde bei 320 nm / 430 nm gemessen und korreliert mit der in den Ansätzen vorhandenen Enzymmenge.
Figur 2: Immunologische Bestimmung der enzymatischen Aktivität von ACE2 in komplexen Lösungen im Kombinationsansatz: Serumproben unterschiedlicher Zeitpunkte eines ARDS Modells wurden mit 0.2 µg/ml ACE2 versetzt und mittels Kombinationsansatz (blaue Balken) und "klassischem" Aktivitätstest in Lösung (graue Balken) vermessen.
Figur 3: Vergleich der matrixabhängigen Signalintensität des Kombinationsansatzes zu ELISA und Aktivitätstest in Lösung.
Figur 4: Menschliche ACE2 Sequenz (SEQ ID NO: 1); Das aktive Zentrum mit dem HEXXH-Zn-Bindungsmotif ist unterstrichen und fett, eine C-terminale Teilsequenz (EFLGIQPTLGPPN; (SEQ ID NO: 2) als Antikörper-Epitop ist als Fettdruck hervorgehoben.

### Beispiel 1: Versuchsdurchführung

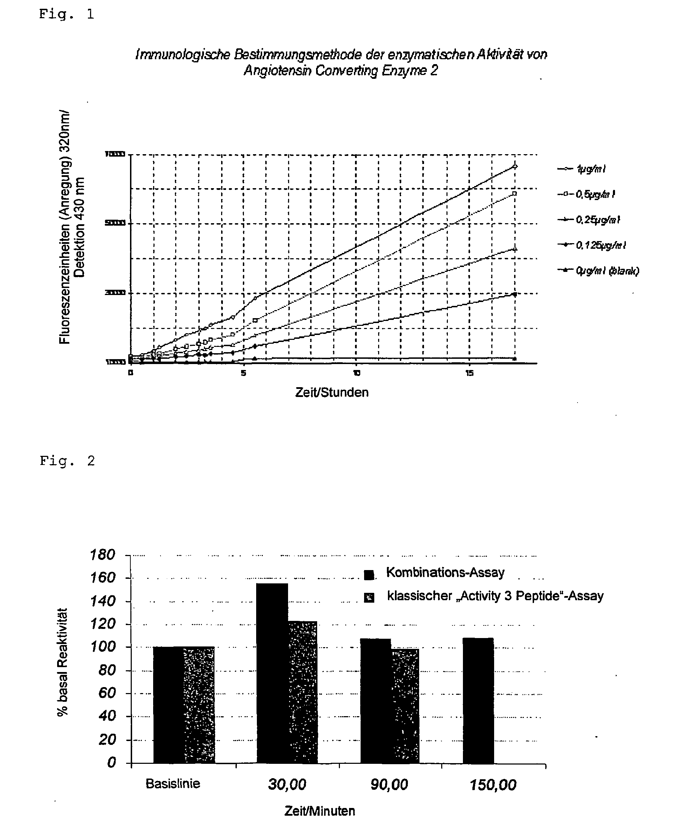

Es wurde eine murine polyklonale ACE2-spezifische Antikörperpräparation an eine 96-Well-Maxisorp-Platte (Nunc) gecoatet und anschließend mit vier Lösungen unterschiedlicher ACE2-Konzentration inkubiert. Nach einem Waschschritt wurde das Peptidsubstrat Mca-Ala-Pro-Lys(Dnp)-OH zugesetzt und die Fluoreszenz über einen Zeitraum von bis zu 17 Stunden gemessen, bei einer Anregungswellenlänge von 320 nm und einer Emissionswellenlänge von 430 nm. Figur 1 zeigt vier Standardkuren unterschiedlicher ACE2-Konzentrationen. Aufgrund der zugrunde liegenden Enzymkinetik erscheint die Produktkonzentration (proportional zur gemessenen Fluoreszenz) in linearem Zusammenhang mit der vorhandenen Enzymmenge, insofern keine Limitierung durch das Substrat auftritt. Die Steigung der Geraden entspricht hierbei den vorhandenen Enzymmengen. Hierbei unterscheiden sich diese Kurven umso deutlicher, je länger die Inkubationszeit angedauert hat, nachdem die Steigung der Geraden im erwähnten Bereich konstant bleibt. Die ebenfalls mitgeführte Blankkurve hebt sich hierbei nicht, auch nicht nach 17stündiger Inkubation, von der Anfangsfluoreszenz ab. Es kann daher der Versuchsansatz je nach gewünschter Empfindlichkeit unterschiedlich lange inkubiert werden.

### Beispiel 2: Absolutmessung einer Enzymmenge in komplexer Matrix

Die vorgestellte Methode soll matrixunabhängige Messungen der ACE2 Aktivität erlauben. In diesem Ansatz wurde die Menge an aktivem ACE2 sowohl mittels des beschriebenen Kombinationstests als auch mithilfe eines "klassischen" Aktivitätstests in Lösung vermessen. Es wurde eine konstante Menge ACE2 in Serumproben zugefügt, welche zu verschiedenen Zeitpunkten eines Sepsis-Modells gezogen wurden. Es wird angenommen, dass die Konzentration an Substanzen, welche die Funktion von ACE2 inhibieren, mit zunehmender Verschlechterung der ARDS Symptome zunimmt.

Während die Messwerte beider Untersuchungsmethoden während der ersten 90 Minuten nahezu dem Ausgangswert (100%) entsprechen, kann zum 150 Minuten Messpunkt ausschließlich im Kombinationsansatz ACE2 Aktivität nachgewiesen werden (Fig. 2). Dies kann dadurch erklärt werden, dass ausschließlich im Kombinationstest ACE2 Inhibitoren durch einen Waschschritt aus dem System entfernt werden und die Aktivitätsmessung nicht beeinflussen. Diese Beobachtung qualifiziert diese Methode zur Serumkomponenten unabhängigen Analytik aktiver Enzyme aus komplexen Lösungen.

### Beispiel 3: Matrixeinflüsse

Die matrixbedingte Beeinflussung der Messsignalintensität (Quenching) des Kombinationsansatzes wurde mit der eines ELISA und der eines Aktivitätstests in Lösung verglichen. Es wurden konstante ACE2 Konzentrationen von 0,5 µg/ml, 0,2 µg/ml, 0,1 µg/ml und 0,05 µg/ml in mehreren Serumverdünnungen beginnend von 1 bis 1:64 gemessen. Zum Vergleich der angeführten Messmethoden wurde die Signalintensität in der höchsten Probenverdünnung von 1:64 auf 100% gesetzt und mit den Resultaten der anderen Verdünnungsstufen verglichen. Mithilfe dieses Vergleichsparameters kann die methodenspezifische matrixbedingte Signalunterdrückung charakterisiert werden (Fig. 3).

Die ELISA Methode unterlag hierbei dem stärksten Matrixeinfluss: Bereits ab den zwei höchsten Verdünnungsstufen wurde eine Signalunterdrückung unter 90% gemessen, welche bei höherer Serumkonzentration weiter rapide zunahm, sodass im konzentrierten Ansatz lediglich eine Restsignalintensität um 20% gemessen werden konnte. Der Aktivitätsansatz in Lösung zeigte den geringsten Matrixeinfluss. Hier konnte man eine deutlich knappere Signalunterdrückung auf 70% in der konzentrierten Probe erkennen. Der Kombinationsansatz zeigt Ähnlichkeiten zu beiden anderen Methoden: Er kennzeichnet sich durch eine deutlich stärkere Matrixabhängigkeit im Vergleich zum Aktivitätsansatz. Jedoch fällt die Signalintensität selbst in der konzentrierten Probe nicht unter 50%.

Diese Methode ermöglicht somit eine ACE2 spezifische Aktivitätsmessung selbst in konzentrierten Serumproben: Durch die immonologische ACE2 spezifische Interaktion des ersten Schrittes wird ausschließlich dieses Enzym in den Versuch eingebracht. Die Messung dessen Aktivität wird im zweiten Schritt nicht durch andere in der Matrix vorhandene Proteasen oder ACE2 Inhibitoren verfälscht.

### Literatur:

1. Danilczyk et al., Circ Res 98, 463-471 (2006).
2. Vickers et al., J Biol Chem 277, 14838-14843 (2002).
3. Ferrario et al., J Am Soc Nephrol 9, 1716-1722 (1998).
4. Imai et al., Nature 436, 112-116 (2005).
5. Kuba et al., Curr Opin Pharmacol (2006).
6. Ye et al., Hypertension 43, 1120-1125 (2004).
7. Huang et al., J Biol Chem 278, 15532-15540 (2003).
8. Guy et al., Biochemistry 42, 13185-13192 (2003).
9. Towler et al., J. Biol Chem 279, 17996-18007 (2004).
10. Burrell et al., Europ Heart J 26, 369-375 (2005).
11. Ishiyama et al., Hypertension 43, 970-976 (2004).

## Patentansprüche

1. Verfahren zur Bestimmung der Aktivität des ACE2, umfassend folgende Schritte:
• zur Verfügung stellen von an einen festen Träger immobilisierten ACE2-bindenden Einheiten, die spezifisch für einen Teil des ACE2 sind, der nicht an der katalytischen Aktivität von ACE2 beteiligt ist,
• Kontaktieren der immobilisierten ACE2-bindenden Einheiten mit einer Probe, die potentiell das ACE2 enthält, wobei das ACE2 von der ACE2-bindenden Einheit gebunden wird, wobei die Probe ausgewählt ist aus Körperflüssigkeiten (wie Vollblut, Serum oder Plasma), homogenisierten fluiden Gewebeproben und Zellkulturüberständen,
• Entfernen der nicht-bindenden Anteile der Probe von dem an die ACE2-bindenden Einheiten gebundenen ACE2 durch Waschen,
• Zufügen eines Substrates des ACE2, das durch die ACE2-Aktivität umgesetzt wird und die Umsetzung ein Signal liefert,
• Messen der Änderung des Signals in einer bestimmten Zeitspanne, wobei die Änderung mit der ACE2-Aktivität korreliert werden kann,
wobei die ACE2-bindende Einheit ein Antikörper, der den C-terminalen Teil der extrazellulären Domäne von natürlichem ACE2 bindet, ist und spezifisch für eine Teilsequenz von mindestens 4 aufeinander folgende Aminosäuren der 362 C-terminalen Aminosäuren von ACE2 ist.

2. Verfahren nach Anspruch 1, **dadurch gekennzeichnet, dass** das Substrat in der Extinktion oder Fluoreszenz verändert wird, wobei vorzugsweise das Substrat einen fluoreszierenden Teil und einen Fluoreszenzquencherteil aufweist die durch die ACE2-Aktivität getrennt werden können.

3. Verfahren nach einem der Ansprüche 1 oder 2, **dadurch gekennzeichnet, dass** das Enzym ACE2 ausgewählt ist aus Membran-gebundenem ACE2 (mACE2) und sekretiertem ACE2 (seACE2).

4. Verfahren nach einem der Ansprüche 1 bis 3, **dadurch gekennzeichnet, dass** die ACE2-bindende Einheit für einen Teil von mindestens 4 Aminosäuren, vorzugsweise 5, 6, 7 oder 8 Aminosäuren, des ACE2 spezifisch, die einen Mindestabstand von 0,5 nm, vorzugsweise 0,6 nm, besonders bevorzugt 0,7 nm oder 0,8 nm, insbesondere 0,9 nm oder 1,0 nm vom aktiven Zentrums aufweist.

5. Verfahren nach einem der Ansprüche 1 bis 4, **dadurch gekennzeichnet, dass** das Substrat ein niedermolekulares Peptid mit einer Länge von 1 bis 30 Aminosäuren, bevorzugt 1 bis 20 Aminosäuren, insbesondere bevorzugt 1 bis 10 Aminosäuren, ist das von ACE2 gespalten werden kann, und an einem zu erwartenden Spaltprodukt der Fluoreszenzteil und an einem anderen zu erwartenden Spaltprodukt der Quencher kovalent verbunden ist, bevorzugt Mca-Ala-Pro-Lys(Dnp)-OH.

6. Verfahren nach einem der Ansprüche 1 bis 5, **dadurch gekennzeichnet, dass** die Probe chromophore und/oder fluoreszierende Verunreinigungen enthält.

7. Verwendung eines Verfahrens nach einem der Ansprüche 1 bis 6 zur *in vitro* Bestimmung von ACE2 Titern in Vollblut, Serum- oder Plasmaproben.

## Claims

1. A method of determining ACE2 activity, comprising the following steps:
- providing ACE2-binding units immobilized on a solid carrier and specific for a part of ACE2 which is not involved in the catalytic activity of ACE2;
- contacting the immobilized ACE2-binding units with a sample which potentially contains the ACE2, wherein the ACE2 is bound by the ACE2-binding unit, wherein the sample is selected from bodily fluids (such as whole blood, serum or plasma), homogenized fluid tissue samples, and cell-culture supernatants;
- removing non-binding portions of the sample from the ACE2 bound to the ACE2-binding units by washing;
- adding a substrate of ACE2 which is converted by the ACE2 activity, with the conversion providing a signal; and
- measuring a change in the signal during a specific period of time, wherein the change can be correlated with the ACE2 activity,
wherein the ACE2-binding unit is an antibody binding the C-terminal portion of the extracellular domain of natural ACE2 and is specific for a partial sequence of at least 4 consecutive amino acids of the 362 C-terminal amino acids of ACE2.

2. The method according to claim 1, **characterized in that** the substrate is altered in the extinction or fluorescence, whereby preferably the substrate has a fluorescent part and a fluorescence-quenching part, which can be separated by the ACE2 activity.

3. The method according to any one of claims 1 or 2, **characterized in that** the enzyme ACE2 is selected from membrane-bound ACE2 (mACE2) and secreted ACE2 (seACE2).

4. The method according to any one of claims 1 to 3, **characterized in that** the ACE2-binding unit is specific for a partial sequence of at least 4 amino acids, preferably 5, 6, 7 or 8 amino acids of the ACE2 which have a minimum distance of 0.5 nm, preferably of 0.6 nm, particularly preferably of 0.7 nm or 0.8 nm, in particular of 0.9 nm or 1.0 nm from the active center.

5. The method according to any one of claims 1 to 4, **characterized in that** the substrate is a low-molecular peptide with a length of from 1 to 30 amino acids, preferably of from 1 to 20 amino acids, particularly preferably of from 1 to 10 amino acids, the peptide being cleavable by ACE2, with the fluorescence part being covalently bound to an expected cleavage product, and the quencher being covalently bound to a different expected cleavage product, preferably Mca-Ala-Pro-Lys(Dnp)-OH.

6. The method according to any one of claims 1 to 5, **characterized in that** the sample contains chromophoric and/or fluorescent contaminations.

7. Use of a method according to any one of claims 1 to 6 for the *in vitro* determination of ACE2 titers in whole blood, serum or plasma samples.

## Revendications

1. Procédé pour déterminer l'activité de l'ACE2, comprenant les étapes suivantes :
• mise à disposition d'unités liant l'ACE2 immobilisées sur un support solide, qui sont spécifiques d'une partie de l'ACE2 qui n'est pas impliquée dans l'activité catalytique de l'ACE2,
• mise en contact des unités liant l'ACE2 immobilisées avec un échantillon qui contient potentiellement l'ACE2, où l'ACE2 est liée par l'unité liant l'ACE2, où l'échantillon est choisi parmi les liquides biologiques (comme le sang total, le sérum ou le plasma), les échantillons tissulaires fluides homogénéisés et les surnageants de culture cellulaire,
• séparation des portions non liantes de l'échantillon de l'ACE2 liée aux unités liant l'ACE2 par lavage,
• addition d'un substrat de l'ACE2, qui est converti par l'activité de l'ACE2 et la conversion délivre un signal,
• mesure de la modification du signal dans une durée déterminée, où la modification peut être corrélée avec l'activité de l'ACE2,
où l'unité liant l'ACE2 est un anticorps qui lie la partie C-terminale du domaine extracellulaire de l'ACE2 naturelle et qui est spécifique d'une séquence partielle d'au moins 4 aminoacides successifs des 362 aminoacides C-terminaux de l'ACE2.

2. Procédé selon la revendication 1, **caractérisé en ce que** le substrat est modifié dans l'extinction ou la fluorescence, où de préférence le substrat comprend une partie fluorescente et une partie extincteur de fluorescence qui peuvent être séparées par l'activité de l'ACE2.

3. Procédé selon l'une des revendications 1 ou 2, **caractérisé en ce que** l'enzyme ACE2 est choisie parmi l'ACE2 liée à la membrane (mACE2) et l'ACE2 sécrétée (seACE2).

4. Procédé selon l'une des revendications 1 à 3, **caractérisé en ce que** l'unité liant l'ACE2 est spécifique d'une partie d'au moins 4 aminoacides, de préférence 5, 6, 7 ou 8 aminoacides, de l'ACE2 qui présente une distance minimale de 0,5 nm, de préférence 0,6 nm, de manière particulièrement préférée 0,7 nm ou 0,8 nm, en particulier 0,9 nm ou 1,0 nm du centre actif.

5. Procédé selon l'une des revendications 1 à 4, **caractérisé en ce que** le substrat est un peptide de faible masse moléculaire d'une longueur de 1 à 30 aminoacides, de préférence 1 à 20 aminoacides, de manière particulièrement préférée 1 à 10 aminoacides, qui peut être clivé par l'ACE2 et est lié par covalence à un produit de clivage à prévoir de la partie fluorescente et à un autre produit de clivage à prévoir de l'extincteur, de préférence Mca-Ala-Pro-Lys (Dnp)-OH.

6. Procédé selon l'un des revendications 1 à 5, **caractérisé en ce que** l'échantillon contient des impuretés chromophores et/ou fluorescentes.

7. Utilisation d'un procédé selon l'une des revendications 1 à 6 pour la détermination *in vitro* de titres d'ACE2 dans des échantillons de sang total, de sérum ou de plasma.
